# EUROPEAN PATENT APPLICATION

(11) **EP 4 324 932 A1**
(43) Date of publication of application: **21.02.2024**
(21) Application number: 22194633.8
(22) Date of filing: 08.09.2022
(51) Int. Cl.: C12Q 1/6806

(54) **NUCLEIC ACID SAMPLE PREPARATION AND ANALYSIS**

(30) Priority: 15.08.2022 US 202263397958 P
(71) Applicant: Koninklijke Philips N.V., 5656 AG Eindhoven (NL)
(72) Inventor: VAN HEMERT, Freek, Eindhoven (NL); WU, Jie, Eindhoven (NL); MACINNES, Alyson, 5656AG Eindhoven (NL); BOUWMAN, Wilbert, 5656AG Eindhoven (NL)
(74) Representative: Philips Intellectual Property & Standards

(57) **Abstract**

Provided is a sample preparation method. The sample preparation method comprises providing a sample comprising nucleic acid and exposing said sample to a first species and a second species. The first species is configured to bind to the nucleic acid in a sequence-agnostic manner. The second species is configured to bind to the nucleic acid in a sequence-specific manner. Further provided is a method of sequencing nucleic acid obtained via the sample preparation method, a method of analyzing said sequenced nucleic acid, and a kit for sample preparation.

## Description

### FIELD OF THE INVENTION

The present disclosure is directed generally to methods and kits for preparing, sequencing and analysing samples comprising nucleic acids.

### BACKGROUND OF THE INVENTION

Next-generation sequencing (NGS) has become a key technology for practicing genetics and genomics. Whole genome sequencing (WGS) by NGS reads the entire content of a human genome (approximately three billion nucleotide pairs) at about 10× to about 30× depth to fully reveal genetics and genomics information.

WGS is currently still quite expensive (€400-1000). Consequently, the sequencing process is often limited to regions of the genome that contain information that is currently (potentially) actionable. The areas of interest can be as large as the entire exome or may just encompass a handful of genes. The former way of sequencing is called whole exome sequencing (WES or WXS) and it covers all the protein coding genes, so about 2% of the entire genome. The latter is called panel sequencing. In both cases the relevant parts of the genome are usually selected using an enrichment technique called Hybrid Selection. Sequencing all the nucleic acid fragments captured during Hybrid Selection enrichment provides information about the targeted genes, like single nucleotide variants (SNVs) or the presence of specific gene fusions. However, genomic information, such as large-scale reorganizations and copy number variants (CNVs), cannot be easily derived from a WES experiment and are even harder to reliably detect using panel sequencing.

Shallow whole genome sequencing (sWGS) samples the entire genome at a reduced depth, making sWGS a reliable method for the detection of large-scale rearrangements, such as CNVs. However, sWGS is typically only at about one one hundredth to one three hundredth of the depth of typical WGS, and may thus be unsuitable for reliably ascertaining single SNVs.

### SUMMARY OF THE INVENTION

The invention is defined by the claims.

There is a continued need for cost-effective methods and kits for obtaining both genetic and genomic insights. The present disclosure is directed to inventive, cost-effective, methods and kits for preparing, sequencing and analyzing a sample to obtain genetic and genomic insights. In particular, the disclosure provides methods for preparing a sample that facilitates, using the prepared sample, genomic and genetic analysis.

According to examples in accordance with an aspect of the invention, there is provided a sample preparation method, the method comprising providing a sample comprising nucleic acid and exposing said sample to a first species and a second species, the first species being configured to bind to the nucleic acid in a sequence-agnostic manner, and the second species being configured to bind to the nucleic acid in a sequence-specific manner.

It is noted that the term "exposing" in this context is intended to mean that the first species and the second species contact the sample in order to enable the first species and the second species to bind to the nucleic acid present in the sample.

The method may enrich nucleic acid in a targeted and untargeted manner in a single sample. The method may be used in preparation to produce a nucleic acid library for sequencing. In some embodiments, the nucleic acid can be a Deoxyribonucleic acid (DNA). In some of other embodiments, the nucleic acid can be a Ribonucleic acid (RNA), such as messenger RNA (mRNA).

The sequence-specific binding of the second species to the nucleic acid permits the targeted enrichment, and the sequence-agnostic binding of the first species to the nucleic acid permits the untargeted enrichment.

The sample preparation method may comprise separating both the nucleic acid bound to the first species and the nucleic acid bound to the second species from a remainder of the sample. This separation step may afford a nucleic acid library.

In some embodiments separating the nucleic acid bound to the first species and the nucleic acid bound to the second species from the remainder of the sample comprises extracting the nucleic acid bound to the first species and the nucleic acid bound to the second species from the remainder of the sample.

Sequence-agnostic, or in other words aspecific, binding of nucleic acid can, in some embodiments, comprise adsorption of the nucleic acid onto a suitable solid phase. In some embodiments, for sequence-agonistic binding, silica beads are used as the solid phase.

The whole process of separating the nucleic acid bound to the first species from the remainder of the sample is termed herein "untargeted enrichment".

The process of binding to specific capture species, in other words probes, may, in some embodiments, comprise hybridization, wherein the nucleic acid anneals or hybridizes to its reverse complementary nucleic acid counterpart.

The whole process of separating the nucleic acid bound to the second species from the remainder of the sample is termed herein "targeted enrichment".

The method may comprise, subsequently to the separating the nucleic acid bound to the first species and the nucleic acid bound to the second species from the remainder of the sample, releasing the nucleic acid from the first species and/or the second species.

The nucleic acid may be released from the first species by "desorption" or "elution". As is known in the art, elution is the process of extracting one material from another by washing with a solvent. As is also known in the art, desorption is the physical process where a previously adsorbed substance is released from a surface.

Alternatively or additionally, the nucleic acid may be released from the second species by heat.

In some embodiments, the nucleic acid may be released from the first species and/or the second species by changing, in other words increasing or decreasing, the pH of the solution.

In some embodiments, the nucleic acid is released by heat and/or pH change, e.g. in combination with mechanical mixing.

In some embodiments, the nucleic acid is released from the second species in a method of melting, dissociating and/or dehybridizing.

In some embodiments, the nucleic acid is released from the first species and/or the second species by exposing the species with the nucleic acid bound thereto to restriction enzymes.

In some embodiments, the first species is in a first amount, and the second species is in a second amount - in other words, the first and second species are provided in a first amount and a second amount, respectively - with the first amount of the first species being selected to bind nucleic acid to obtain 0.01× to 1× coverage of the nucleic acid in the sample and the second amount of the second species being selected to bind to the nucleic acid to obtain at least 10× coverage, preferably 20x or more coverage of the specific sequences in the sample with which the second species binds.

Alternatively or additionally, the second amount of the second species relative to the first amount of the first species is such that the coverage obtained of the nucleic acid in the sample is 10× or more higher for nucleic acid bound by the second species compared to nucleic acid bound by the first species.

In this manner, enrichment by the first species and the enrichment by the second species may be balanced. Balance may be achieved by tuning the amount of the first species and/or the second species, or the relative affinities of the first species and the second species for binding nucleic acid in a sequence-agnostic manner or binding targeted nucleic acid sequences, respectively.

In some embodiments, balance may be achieved by adjustment of amount of the first species relative to a density of capture species, i.e. capture probes, supported on a solid support, such as silica, e.g. silica beads. For example, silica beads and hybrid capture probe-covered beads may be mixed in the carefully tuned amounts, so that the desired coverage is achieved from both species. In an embodiment, this would result in 0.01× to 1× coverage of the nucleic acid and 10× or more coverage, such as 20x or more coverage or 100× or more coverage of the specific sequences. In another complementary or alternative embodiment, the method could be used to obtain 20-30x coverage of all of the nucleic acid molecules (for whole genome sequencing) and targeted coverage of 100× or more of the targeted sequence or sequences.

In some embodiments, said exposing of the sample to the first species and the second species comprises an initial contacting of the sample with the first species and a further initial contacting of the sample with the second species, wherein the initial contacting is concurrent with the further initial contacting.

The "initial contacting" and the "further initial contacting" being concurrent with each other is intended to mean that the sample is exposed to, in other words contacted by, the first species and the second species added thereto at the same time.

For example, both of the first and second species may be supplied in the same kit, wherein the kit enables simultaneous combination of the first and second species with the sample.

Performing initial contacting with the first species and the second species concurrently may reduce the time to carry out the described method, which is one of the advantages of the present disclosure. For example, since only a single incubation step would be required to allow binding to both of the first and second species, this would reduce the required time.

Such a single incubation step may also mean less variation between replicate occurrences of the method because the number of experimental variables is reduced. Concurrent contacting may also facilitate attaining the desired balance through the tuned relative amounts of the first and second species and/or their relative affinities for binding nucleic acid in a sequence-agnostic manner or binding targeted nucleic acid sequences, in other words through a ratio between the two modes of binding.

In some embodiments, the first species comprises a first solid phase, and/or the second species comprises a second solid phase.

In some embodiments, one or more of the species is a solid phase.

A solid phase can be readily separated from the fluid comprising the nucleic acid sample.

In some embodiments, the solid phase can be separated from the sample by centrifugation.

In some embodiments, the solid phase is magnetic and can be separated from the sample using magnets.

The binding of the nucleic acid to the one or more solid phases may take place in a column or on a surface.

In some embodiments, one or both of the first and second solid phases comprise(s) beads. Typically, one or more of the solid phases are beads.

Beads may be separated from the sample by centrifugation.

In some embodiments, the beads are magnetic and may be separated from the sample using magnets.

In some embodiments, the second species comprises at least one capture species, each of the at least one capture species being configured to selectively bind to a specific nucleic acid sequence of the nucleic acid.

The specific capture sequence may be the reverse complement of the target nucleic acid sequence (single stranded). The single stranded reverse complement nucleic acid may bind to its complementary (target) partner.

Binding of one or more specific nucleic acid sequences to the second species may be termed "hybrid capture" or "hybrid capture based target enrichment".

In some embodiments, the specific capture sequence may comprise peptide nucleic acid (PNA). PNA is a molecule similar to nucleic acid that can bind to a nucleic acid sequence in the same manner as reverse complementary nucleic acid can bind to a nucleic acid sequence. PNA has a protein backbone and may be more stable. PNA may bind to nucleic acid more tightly, in other words with higher affinity.

A second species comprising PNA may be preferable to a second species comprising DNA.

In some embodiments, the at least one capture species comprises a plurality of capture species that are different from each other, each of the plurality of capture species being configured to bind to a specific nucleic acid sequence different from that bound by the other capture species of the plurality of capture species.

The plurality of sequence-specific capture species may enable enrichment of a plurality of specific nucleic acid sequences in a single step.

The respective concentrations of each of the plurality of capture species may differ from each other.

This may result in balanced target enrichment between the specific sequence bound by one of the plurality of capture species and a different specific sequence bound by another of the plurality of capture species.

In some embodiments, each of the plurality of capture species is supported on beads, such as silica beads.

In some further embodiments, the solid phase may comprise a first solid phase, e.g. a first set of silica beads, on which a first capture species of the plurality of capture species is supported, and a second solid phase, e.g. a second set of silica beads, on which a second capture species of the plurality of capture species is supported.

Alternatively, the first and second captures species may be supported on a common solid phase, such as on the same silica bead surface.

In another aspect of the invention, there is provided a method of sequencing one or more samples of nucleic acid prepared via the sample preparation method according to any of the embodiments described herein.

In some embodiments, sequencing is performed using a next-generation sequencer (NGS), such as a next-generation sequencer, like Ion S5 produced by Illumina. NGS leverages sequencing by synthesis to track the addition of labelled nucleotides as the nucleic acid chain is copied. It may be massively parallel and generates large amounts of DNA sequence data.

The method of sequencing may comprise targeted sequencing of the nucleic acid that bound to the second species.

Alternatively or additionally, the method of sequencing may comprise shallow whole genome sequencing (sWGS) of the nucleic acid that bound to the first species.

The method of sample preparation and sequencing of the nucleic acid bound to the second species may be termed hybrid capture and sequencing. The coverage of sequencing may be determined by the sample preparation method and the balance of the first species and the second species.

In some embodiments, the amount of available first species would allow shallow whole genome sequencing (sWGS) to be carried out. In other embodiments, the amount of the first species would allow whole genome sequencing (WGS) to be carried out.

In some embodiments, the amount of the second species facilitates targeted sequencing. The balance of the first species and the second species may enable the combination of sWGS and targeted sequencing in a single experiment.

In some embodiments, the combination of sWGS and WGS can be used.

In some embodiments, the method comprises applying a first identifier, such as a first barcode, to the whole genome nucleic acid library, and applying a second identifier, such as a second barcode, to the target sequencing library.

Applying identifiers, such as barcodes, may assist in managing libraries containing more than one sample. For example, a nucleic acid sequencer may be capable of sequencing 1000 million reads. In some embodiments, the sequencing method only utilizes 100 million reads per sample, so for instance 10 samples (optionally from 10 patients) may be sequenced in parallel and the identifiers may be used to distinguish the sequence data of the different samples after sequencing. Typically, the nucleic acid identifiers are attached to the reads before the sample or samples enter the sequencer. Typically, the nucleic acid in each sample comprises a distinct nucleic acid identifier.

In still another aspect there is provided a method of analyzing nucleic acid sequence data, which nucleic acid sequence data is obtained by the method of sequencing according to any of the embodiments described herein.

In at least some embodiments, the nucleic acid sequence data to be analyzed comprise genetic and genomic information.

In yet another aspect there is provided a computer program code that is configured, when said computer program is run on one or more processors, to cause said one or more processors to implement the method of analyzing the nucleic acid sequence data according to any of the embodiments described herein.

In a further aspect there is provided a kit for sample preparation, the kit comprising a first species and a second species for exposing to a sample comprising nucleic acid, the first species being configured to bind to the nucleic acid in a sequence-agnostic manner, and the second species being configured to bind to the nucleic acid in a sequence specific manner.

The first species and the second species may be as described above in relation to the sample preparation method.

More generally, embodiments described herein, in particular in relation to the sample preparation method, may be applicable to the kit, and embodiments described herein in relation to the kit may be applicable to the methods, and in particular the sample preparation method.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is an illustration of an exemplary combined method of sample preparation, sequencing and analysis; and
Fig. 2 shows a hypothetical result of a sequencing method according to an example.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is a sample preparation method. The sample preparation method comprises providing a sample comprising nucleic acid and exposing said sample to a first species and a second species. The first species is configured to bind to the nucleic acid in a sequence-agnostic manner. The second species is configured to bind to the nucleic acid in a sequence-specific manner. Further provided is a method of sequencing nucleic acid obtained via the sample preparation method, a method of analyzing said sequenced nucleic acid, and a kit for sample preparation.

The invention provides various embodiments of methods and kits for obtaining genetic and genomic insights. The present disclosure is directed to inventive methods and kits for preparing, sequencing and analyzing a sample to obtain genetic and genomic insights.

Typically, WGS at 30x depth or more is necessary to obtain genetic and genomic insights. The inventors identified a method for preparing, sequencing and analyzing a sample to obtain genetic and genomic insights, wherein 30x depth for the entire genome is not necessary. The method is inexpensive and provides for enrichment of DNA for targeted sequencing and shallow whole genomic sequencing (sWGS).

It is known in the art to use whole exome sequencing (WES) to identify copy number variants. sWGS may be advantageous compared to WES due to more uniform coverage of the entire whole genome rather than just the protein-coding genes as with WES.

The method of the disclosure combines target enrichment sample preparation with untargeted enrichment in a balanced manner to generate a sequencing library that contains enough fragments from specific genes of interest to cover them at a depth of 30x or higher while we cover the rest of the genome uniformly at a depth of about 0.1×. This enables analysis to identify information about the genetics of the genes of interest (including, for example, SNVs) without sacrificing the ability to gain insights into the large-scale structure of the genome.

The whole genome nucleic acid library and the target sequencing library may be labelled with identifiers prior to sequencing, to enable separate analysis for either CNVs or SNVs.

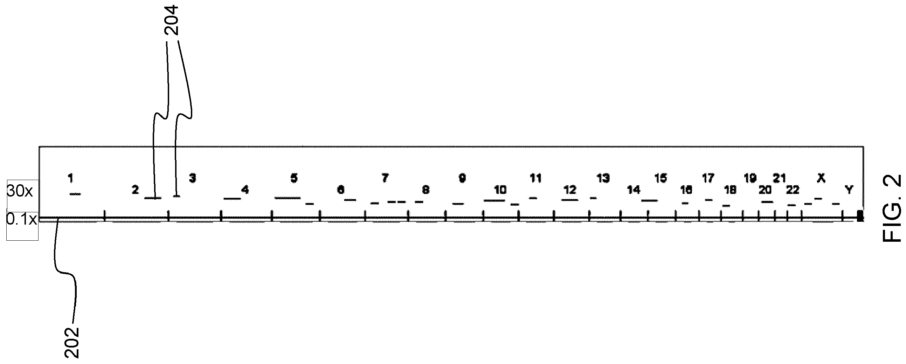

Fig. 2 shows a hypothetical result of such a sequencing experiment.

A combined panel and sWGS experiment may, by combining genetics and genomics, yield valuable insights (Jiao et al., The combination of whole-exome sequencing and copy number variation sequencing enables the diagnosis of rare neurological disorders, Clin. Genet. 2019; 96(2): 140-150).

The method of the disclosure may be used with and integrated into existing protocols like NEBNext Direct Target Enrichment, as well as protocol aspects such as Illumina's on-bead fragmentation. More protocols and protocol aspects would be apparent to the skilled person (Kozarewa et al., Overview of Target Enrichment Strategies, Current Protocols in Molecular Biology, 112(1): 7.21.1-7.21.23).

### Sample preparation

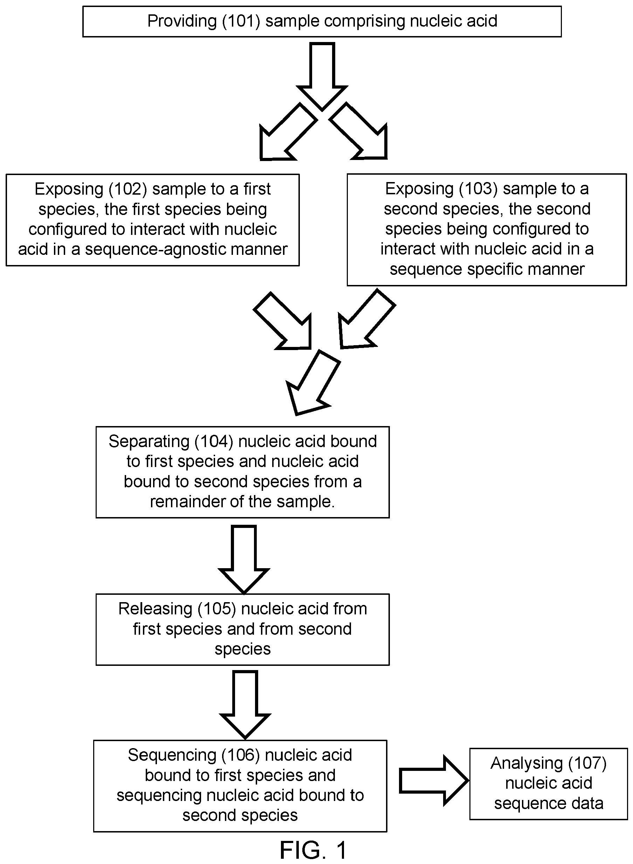

The sample preparation method of the disclosure is directed to enrichment of nucleic acid in a sample. Referring to Fig. 1, the method comprises providing 101 a sample comprising nucleic acid.

The sample may be any sample comprising nucleic acid. The sample may be obtained from a mammal, optionally a human. In some embodiments, the sample is a bodily fluid, optionally blood. In some embodiments, the sample is used directly in the method. In alternative methods, the sample is processed prior to applying the method of the disclosure.

In some embodiments, the nucleic acid in the sample is from the same organism from which the sample is taken. In alternative embodiments, some or all of the nucleic acid in the sample is from a different organism to that from which the sample is taken. The nucleic acid in the sample may be mammalian, optionally human. The nucleic acid in the sample may be viral or bacterial. In some embodiments, the nucleic acid in the sample comprises a genome. Optionally, the nucleic acid comprises a human genome. In some embodiments, the sample comprises tumour nucleic acid, e.g. tumour DNA, such as circulating tumour DNA. Optionally, the sample comprises the genome of a tumour cell. Optionally the sample comprises circulating tumour nucleic acid. In some embodiments, the sample is blood, optionally comprising tumour nucleic acid.

The method of the disclosure is directed to enriching nucleic acid in a targeted and untargeted manner in a single sample in preparation to produce a nucleic acid library for sequencing. Referring to Fig. 1, the method comprises exposing 102 the sample to a first species and exposing 103 the sample to a second species.

The first species is configured to bind to the nucleic acid in a sequence-agnostic manner. The nucleic acid may adsorb to the first species. The first species binds to nucleic acid regardless of the sequence of the nucleic acid. In some embodiments, the first species comprises silica. In a preferred embodiment, the first species is silica beads, in particular unfunctionalised silica beads.

The second species is configured to bind to the nucleic acid in a sequence-specific manner. In some embodiments, the second species binds to the nucleic acid by hybridization. In some embodiments the second species anneals to the specific nucleic acid sequences. Optionally, the second species comprises a nucleic acid sequence that is the reverse complementary counterpart to the target nucleic acid sequence. In some embodiments, the second species is peptide nucleic acid (PNA). PNA is an artificial molecule comprising a peptide backbone. It binds to reverse complementary nucleic acid. PNA is more stable than nucleic acid and binds the target nucleic acid more tightly. Therefore, a second species comprising PNA may be advantageous.

In some embodiments, the first species and the second species comprise a common element, such as silica. The common element may serve as a support for the second species, e.g. capture species, and the common element may further define the first species.

In other words, the common element may be a sequence agnostic species, and the second species may be supported by a portion of the common element, with the second species comprising a moiety that selectively binds to a specific nucleic acid sequence. In one embodiment, the common element is a silica bead supporting nucleic acid or PNA molecules specific for the target sequences but with the silica bead comprising portion(s) which do not support the nucleic acid of PNA molecules and hence are available for non-specific binding.

In some embodiments, the first species is separate to the second species. For example, the first species is in the form of silica beads, e.g. unfunctionalised silica beads, and the second species is in the form of capture species, e.g. nucleic acid or PNA molecules, supported on further silica beads.

In some embodiments, such as that shown in Fig. 1, the method comprises separating 104 the nucleic acid bound to the first species and the nucleic acid bound to the second species from the remainder of the sample. In some embodiments, the first species and the second species are separated from the remainder of the sample, wherein nucleic acid remains bound to the first species in a sequence agnostic manner and the nucleic acid remains bound to the second species in a sequence specific manner during separation of the species from the remainder of the sample. In some embodiments, the first species and/or the second species with bound nucleic acid are taken out of the sample. Optionally, the first species and/or the second species are magnetic and a magnet may be used to separate one or both species with bound nucleic acid from the remainder of the sample.

In alternative embodiments, the first species and the second species are washed to separate the nucleic acid from the sample and replace any fluid surrounding the beads with the wash buffer. The nucleic acid remains bound to the first species and the second species during washing. The first species and/or the second species may be configured as a column, over which the sample is run. Following adsorption, a wash buffer may be run over the column.

In some embodiments, such as that shown in Fig. 1, the method comprises releasing 105 the nucleic acid from the first species and/or from the second species. Release of the nucleic acid from the first species or from the second species may be termed "extraction" or "nucleic acid extraction". In some embodiments, the nucleic acid is released from the first species and the second species. Optionally the nucleic acid is released from the first species and the second species concurrently. Nucleic acid may be released from the first species by "desorption" or by "elution". In some embodiments, the nucleic acid is eluted or desorbed by buffer exchange. Nucleic acid may be released from the second species by heat. Optionally, the nucleic acid is released from the second species in a method of melting, dissociating and/or dehybridizing.

The method of the disclosure may comprise enriching nucleic acid on the first species to obtain 0.01× to 1× coverage of the nucleic acid in the sample. Optionally, coverage of the nucleic acid in the sample is 0.05x to 1×, or 1×. Enrichment of nucleic acid on the first species may be sufficient for shallow whole genome sequencing. Enrichment of nucleic acid on the first species may enable the identification of copy genetic alterations. The genetic alterations may be copy number variations.

The method of the disclosure may comprise enriching nucleic acid on the second species to obtain 10× or more coverage of the specific sequences in the sample with which the second species binds. In some embodiments, the second species obtains 20-100x coverage of the specific sequences, optionally 20-50× coverage. In some embodiments, the second species obtains 20-30× coverage of the specific sequences. In alternative embodiments, the second species obtains 100× or more coverage of the specific sequences. Enrichment of nucleic acid on the second species may be sufficient for targeted sequencing. Enrichment of nucleic acid on the second species may enable the identification of genetic alterations. The genetic alterations may be single nucleotide variations.

In some embodiments, the method comprises balancing enrichment by the first species and enrichment by the second species. Balancing between the first species and the second species may be achieved by tuning the amount of the first species and/or the amount of the second species. Balancing may be achieved by tuning the relative affinities of the first species and/or the second species for binding nucleic acid. In some embodiments, balancing may be achieved by tuning the amount and affinities of the first species and/or the second species.

In an alternative embodiment of the disclosure, balancing may obtain 20-30× coverage of the whole nucleic acid and 100× or more coverage for the specific nucleic acid sequence or sequences. Optionally, the sample comprises tumour nucleic acid.

In some embodiments, the initial contacting of the sample with the first species is concurrent with a further initial contacting of the sample with the second species. In some embodiments, the first species and the second species may be supplied in the same kit, wherein the kit enables simultaneous combination of the first and second species with the sample. Concurrent contacting may reduce the time taken to perform the method of the disclosure compared to the method wherein the sample is first contacted with the first species and subsequently contacted with the second species. Concurrent contacting may only require a single incubation. Concurrent incubation reduces variation in the experimental conditions, in particular due to the single incubation step, compared to the method wherein the sample is first contacted with the first species and subsequently contacted with the second species. Therefore, concurrent contacting may reduce variation between replicate occurrences of the method.

In some embodiments, the sample preparation method of the disclosure is utilized on multiple different samples. The multiple different samples may be from the same source or from difference source. Optionally, the multiple different samples are taken from different patients.

The sample may comprise any nucleic acid. Typically the nucleic acid is a naturally occurring nucleic acid. The nucleic acid may be from any organism.

In some embodiments, the nucleic acid is DNA.

In some embodiments, the nucleic acid is RNA. Optionally, the nucleic acid is messenger RNA (mRNA).

### Sequencing

As shown in Fig. 1, the present disclosure further contemplates sequencing 106 the one or more nucleic acid samples prepared according to the above-described sample preparation method. The sequencing 106 provides nucleic acid sequence data.

Methods of sequencing nucleic acid are known in the art. In some embodiments, DNA sequencing occurs via the Sanger method. Alternatively, high-throughput sequencing or next-generation sequencing methods may be used for DNA sequencing. Typically the method of sequencing is next-generation sequencing (NGS). NGS is a form of massively parallel sequencing technology. In some embodiments, NGS may sequence 1000 million reads simultaneously.

In some embodiments, RNA is translated into DNA and DNA sequencing takes place to ascertain the sequence of the DNA. Based on the DNA sequence, the original RNA sequence can be ascertained by complementary base pair analysis. In alternative embodiments, RNA may be sequenced directly. In some embodiments, RNA is sequenced by NGS.

The coverage of sequencing is determined by the sample preparation method of the disclosure. In some embodiments, nucleic acid enrichment on the first species enables shallow whole genome sequencing (sWGS). The depth of sWGS may be at least about 0.01×, optionally 0.01×, 0.1× or 1× of the genome.

In some embodiments, nucleic acid enrichment on the second species enables targeted sequencing. Targeted sequencing may provide coverage of one or more genes of interest at a depth of 10× or higher. Optionally, the depth of targeted sequencing is 20x or higher, 30x or higher, 40x or higher, 50× or higher, 60x or higher, 70x or higher, 80x or higher, 90x or higher, or 100× or higher.

In some embodiments, sequencing can be conducted on 1000 million reads simultaneously. The method of the disclosure may only require 100 million reads per sample. Therefore, multiple samples may be sequenced simultaneously. Typically, where multiple samples are sequenced simultaneously, the method comprises applying an identifier to the nucleic acid library from each sample. A different identifier may be applied to each sample. The identifier is applied to the nucleic acid in each sample prior to the sample or samples entering the sequencer for sequencing. The identifier may be a barcode.

### Analysis

Referring again to Fig. 1, the sequence data obtained from the enriched nucleic acid may be analysed 107.

The sequence data may be reconstructed to produce the original genome of the sample. Aberrations from the separately sequenced fragments may be identified by comparing the sequence data of the sample to a template sequence known in the art. In some embodiments, one or more of the samples may be a template sequence to which the other samples are compared.

The sequence data to be analysed may contain genetic and genomic information.

Typically, analysis is conducted using a computer program code configured, such that when it is run on one or more processors, it implements a method to analyse the nucleic acid sequence data, e.g. the DNA sequence data.

The analysis may identify single nucleotide variations. In particular, the enrichment from the second species may enable the identification of single nucleotide variations in the sample.

The analysis may identify copy number variations in one or more samples. In particular, enrichment from the first species may enable the identification of copy number variations.

The results of analysis may be used to identify genetic abnormalities in one or more samples, optionally in one or more patients. The one or more genetic abnormalities identified may result in diagnosis and or treatment with one or more therapies.

The disclosure is also directed to a computer program comprising computer program code that is configured, when said computer program is run on one or more processors, to cause said one or more processors to implement the method of analyzing nucleic acid. Functions implemented by a processor may be implemented by a single processor or by multiple separate processing units which may together be considered to constitute a "processor". Such processing units may in some cases be remote from each other and communicate with each other in a wired or wireless manner.

A computer program may be stored/distributed on a suitable medium, such as an optical storage medium or a solid-state medium supplied together with or as part of other hardware, but may also be distributed in other forms, such as via the Internet or other wired or wireless telecommunication systems.

### Kit

The disclosure is also directed to a kit for sample preparation.

The kit may comprise a first species and a second species for exposing to a sample comprising nucleic acid. The first species is configured to bind to the nucleic acid in a sequence-agnostic manner, and the second species is configured to bind to the nucleic acid in a sequence specific manner.

In some embodiments, the kit comprises the first species and the second species in a sample well. The sample may be introduced into the sample well and a single incubation step may occur.

### Examples

### Example 1

The disclosure comprises a hybrid selection-based sample preparation kit, which constitutes a panel. The kit comprises a second species that enriches fragments for specific regions of the genome. The kit also comprises a first species that non-specifically enriches a random pool of fragments in the sample. The non-specific - or sequence-agnostic - first species provides shallow coverage of the whole genome.

The disclosure comprises an algorithm tailored to make optimal use of the sequencing results to derive genetic and genomic information.

Fig. 1 provides an illustration of the combined method of sample preparation, sequencing and analysis. In step 101 a sample is provided, for instance a FFPE tissue slide, from which nucleic acid extraction occurs, according to the method of sample preparation of the disclosure. The nucleic acid may be any nucleic acid. Typically, the nucleic acid is DNA. The method of nucleic acid extraction may result in a tube of purified nucleic acid. During sample preparation the first species and the second species are added 102, 103 to the sample in a predetermined ratio. The nucleic acid in the sample binds to the species. The species to which nucleic acid is bound may be separated 104 from the remainder of the sample. Optionally, the species and nucleic acid is separated by purifying and transferring the species to another tube. The nucleic acids may be released 105 from the beads and a sequencing library is thereby afforded. The sequencing library is loaded onto a sequencer chip and a sequencing process is executed 106.

Fig. 2 is a hypothetical result of a sequencing method according to the disclosure. The sequence data generated by the sequencing 106 shown in Fig. 1 may be used for sequence data analysis 107. Target enrichment and shallow whole genome library may be mapped to a human reference genome. The solid line 202 at 0.1× coverage shows the coverage of the shallow whole genome sequencing (sWGS) component. The sWGS is homogeneous over the whole genome at an average depth of 0.1×. The higher lines 204 at about 30x coverage show the genome of interest, captured using target enrichment. Specific areas of the genome are sequenced at a depth of 30x. More and smaller target regions can be distributed through the genome.

### Example 2

In a further example, the disclosure is directed to target enrichment probes that constitute a second species according to the disclosure. Nucleic acid capture beads that bind non-specifically to any nucleic acid constitute a first species according to the invention. Binding of nucleic acid to the first species and to the second species enriches the nucleic acid in the sample to generate a sequencing library.

The first species and the second species are two different types of beads. The first species is silica beads that sequence-agnostically bind to nucleic acid. The second species are beads comprising probes that bind to specific nucleic acids in a sequence-specific manner.

### Example 3

In one example, the first species is silica beads, e.g. unfunctionalised silica beads, and the second species is in the form of capture species, i.e. target enrichment probes, supported on further silica beads.

Nucleic acid sequence-agnostically binds to the silica bead of the first species and sequence-specifically binds to the capture species of the second species.

### Example 4

The nucleic acid in the sample may be tagged with a barcode. In some embodiments, the nucleic acid bound to the first species is tagged with one barcode and the nucleic acid bound to the second species is tagged with a different, second barcode.

The different barcodes may enable different analysis of the sequenced reads. For example, the nucleic acid bound to the first species may be utilized for a copy number variation calling pipeline and the nucleic acid bound to the second species may be utilized for single nucleotide variant calling.

### Example 5

An exemplary embodiment of the disclosure is directed to shotgun metagenomics for global species identification and anti-microbial resistance gene panel.

The first species of the disclosure enables global species identification through low depth coverage of the entire nucleic acid in the sample. This enables identification of bacteria at the species level and differentiation between closely related bacterial species. The amount and affinity of the first species results in 0.01× to 1× coverage of the nucleic acid in the sample.

The second species of the disclosure is configured to bind to nucleic acid sequences known to have single nucleotide polymorphisms (variants) associated with anti-microbial resistance. The amount and affinity of the second species results in at least about 10× depth coverage, optionally at least about 20x depth coverage.

The balance of the first species and the second species allows species identification and anti-microbial resistance detection, respectively, in a cost-effective manner.

The first species and the second species are magnetic silica beads. The second species comprise target enrichment probes. Tuning of the enrichment probe concentration on the beads results in optimal sequencing-ratio for species identification and anti-microbial resistance detection.

### Example 6

In an exemplary embodiment of the disclosure, more than 2 sequencing applications may be combined in a single step.

For example: 1) sWGS (0.1-1× coverage). 2) Targeted panel for pharmacogenetics (20x coverage). 3) Targeted tumor panel (100× coverage).

In this example, the second species comprises two different capture species, wherein the first capture species is configured to bind to a different specific sequence from that bound by the second capture species.

The concentrations of the different capture species may differ from each other, resulting in balanced target enrichment between the specific sequence bound by the first capture species and the different specific sequence bound by the second capture species.

In one embodiment, the second species comprises two different capture species, with each of the capture species being supported on respective solid phases, e.g. beads. In this embodiment, the first species is defined by a solid phase, e.g. beads, separate from the solid phases supporting the two different capture species.

In another embodiment, the second species comprises two different capture species, with both of the capture species being supported on a common solid phase, e.g. beads. In this embodiment, the first species is defined by a solid phase, e.g. beads, separate from the solid phase supporting the two different capture species.

In a further example, the second species comprises three capture species which are all different from each other. The first capture species is configured to bind to a different specific sequence from that bound by the second and third capture species. The second capture species is configured to bind to a different specific sequence from that bound by the first and third capture species. The third capture species is configured to bind to a different specific sequence from that bound by the first and second capture species.

In such an example, each of the capture species can be supported on a respective solid phase, e.g. beads. In this embodiment, the first species is defined by a solid phase, e.g. beads, separate from the solid phases supporting the three different capture species.

The method may allow genomic analysis using shallow whole genome sequencing, a targeted panel to study the pharmacogenetics (germline related) and the targeted tumor panel for in-depth analysis of tumor genes.

Another example of combining multiple applications is combining Non-invasive prenatal test (NIPT) testing together with a targeted panel e.g. pharmacogenetic panel. The NIPT test uses sWGS and a pharmacogenetic specific panel. Two types of silica/beads can be used in which one is a-specific and one is specific using capture probes. The sWGS allows the detection of copy number variations and the deep sequencing of the targeted panel allows the detection of pharmacogenetic aberrations.

### Example 7

In one embodiment, transcriptomics and a targeted panel for fusion genes, single-nucleotide variants, alternative splicing, or pathway analysis may be combined. RNAseq and DNAseq can be combined, wherein the relative capture abundance is tuned as set out in the disclosure, optionally by tuning the amounts and/or affinities of the capture probes of the second species. The method of the disclosure may enable the detection of fusions, detection of alternative splicing variants, single-nucleotide variants, or for more robust pathway analysis (for example when target genes are expected to be expressed at a lower level). The use of barcodes allows for the distinction between differential gene expression and the targeted panel.

To overcome the problem of exhausting the starting sample by using the first species and thereby introducing bias for the second species, different concentrations of the first species and the second species may be used. For example, for the transcriptomics 10% of the sample is captured by the first species and using the second species for the targeted panel 90% is captured. In this manner minimal bias will be introduced. Alternatively, the second species could be constructed in such a way that we are compensating for the reduced concentration after the first capture.

One example of targeted RNA binding is the Illumina targeted RNA sequencing library preparation kit (https://www.illumina.com/techniques/sequencing/rna-sequencing/targeted-rna-seq.html).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A sample preparation method, the method comprising:
providing a sample comprising nucleic acid; and
exposing said sample to a first species and a second species, the first species being configured to bind to the nucleic acid in a sequence-agnostic manner, and the second species being configured to bind to the nucleic acid in a sequence-specific manner.

2. The sample preparation method according to claim 1, comprising separating the nucleic acid bound to the first species and the nucleic acid bound to the second species from a remainder of the sample.

3. The sample preparation method according to claim 2, comprising, subsequently to the separating the nucleic acid bound to the first species and the nucleic acid bound to the second species from the remainder of the sample, releasing the nucleic acid from the first species and/or from the second species.

4. The sample preparation method according to any of claims 1 to 3, wherein the first species is in a first amount, and the second species is in a second amount, the first amount of the first species being selected to bind to nucleic acid to obtain 0.01× to 1× coverage of the nucleic acid in the sample and the second amount of the second species being selected to bind to the nucleic acid to obtain 10× or more coverage of the specific sequences in the sample with which the second species binds.

5. The sample preparation method according to any of claims 1 to 4, wherein said exposing of the sample to the first species and second species comprises:
an initial contacting of the sample with the first species, and
a further initial contacting of the sample with the second species, wherein the initial contacting is concurrent with the further initial contacting.

6. The sample preparation method according to any of claims 1 to 5, wherein the first species comprises a first solid phase, and/or the second species comprises a second solid phase, wherein optionally one or both of the first and second solid phases comprise(s) beads.

7. The sample preparation method according to any of claims 1 to 6, wherein the second species comprises at least one capture species, each of the at least one capture species configured to selectively bind to a specific nucleic acid sequence of the nucleic acid.

8. The sample preparation method according to claim 7, wherein the at least one capture species comprises a plurality of capture species that are different from each other, each of the plurality of capture species being configured to bind to a specific nucleic acid sequence different from that bound by the other capture species of the plurality of capture species.

9. A method of sequencing one or more samples of nucleic acid prepared via the sample preparation method according to any of claims 1 to 8.

10. The method according to claim 9, wherein the method of sequencing comprises targeted sequencing of the nucleic acid that bound to the second species.

11. The method according to claim 9 or claim 10, wherein the method of sequencing comprises shallow whole genome sequencing, sWGS, of the nucleic acid bound to the first species.

12. A method of analysing nucleic acid sequence data, which nucleic acid sequence data is obtained by the method of any of claims 9 to 11.

13. The method according to claim 12, wherein the nucleic acid sequence data comprise genetic and genomic information.

14. A computer program comprising computer program code that is configured, when said computer program is run on one or more processors, to cause said one or more processors to implement the method according to claim 12 or claim 13.

15. A kit for sample preparation, the kit comprising a first species and a second species for exposing to a sample comprising nucleic acid, the first species being configured to bind to the nucleic acid in a sequence-agnostic manner, and the second species being configured to bind to the nucleic acid in a sequence specific manner.
